# EUROPEAN PATENT APPLICATION

(11) **EP 4 840 502 A1**
(43) Date of publication of application: **18.11.2026**
(21) Application number: 24855088.1
(22) Date of filing: 03.06.2024
(51) Int. Cl.: A61N 1/40, A61B 18/14, A61N 1/32, A61N 1/06, A61N 1/08, A61N 1/36, A61N 1/04, A61B 18/00

(54) **BEAUTY TREATMENT DEVICE**

(30) Priority: 10.01.2024 KR 20240004236
(71) Applicant: Amolifescience Co., Ltd., Seoul 06527 (KR); Minimizing Co., Ltd., Seongnam-si, Gyeonggi-do 13595 (KR)
(72) Inventor: JEONG, Sangdong, Seoul 06527 (KR); PARK, Jinpyo, Seoul 06527 (KR); LEE, Jeongho, Seongnam-si, Gyeonggi-do 13595 (KR)
(74) Representative: RGTH
(86) International application number: PCT/KR2024/007574
(87) International publication number: WO 2025/150632

(57) **Abstract**

Disclosed is a cosmetic treatment device that destroys fat cells by locally suctioning the skin to be treated and sequentially applying radio frequency current and high voltage pulses thereto. The cosmetic treatment device according to the present invention includes a main body portion and a suction treatment portion coupled to the main body portion and configured to suction the skin of a user and apply electrical stimulation thereto, wherein the suction treatment portion includes a suction nozzle made of a resin material and configured to divide the treatment space and a pair of electrodes disposed inside the suction nozzle to face each other while spaced apart from each other, and the pair of electrodes have a configuration in which electrode cores made of a resin material have surfaces plated with a conductive metal to form metal conductive layers.

## Description

### [Technical Field]

The present invention relates to a cosmetic treatment device, and more particularly, to a cosmetic treatment device that improves skin elasticity by destroying unnecessary fat cells by suctioning the skin using a negative pressure and delivering radiofrequency energy and high voltage pulses to the fascial plane.

### [Background Art]

The elasticity of not only the epidermis, but also the dermis and the fascial plane, of the skin may decrease starting in one's late 20s when aging actually starts. This causes sagging skin and wrinkles. Already known methods of improving skin elasticity include using home care firming creams to get rid of a sagging double chin and reduce buccal fat and stimulating specific areas of the skin using gua sha or the like.

In recent years, as non-invasive treatment methods that can simultaneously improve elasticity of sagging skin and reduce fat, a method of removing fat layers by applying radiofrequency energy to the skin (a method of inducing the death of fat cells using thermal energy generated when radiofrequency current of 1 MHz or more is passed into the skin), a method of enabling electroporation by applying high voltage pulses to the skin, etc. have been used.

For reference, electroporation is a phenomenon in which, when strong, short electrical pulses are applied to a cell membrane, pores are formed in a surface of the cell membrane due to an electric potential difference between both ends of the cell membrane. The electroporation technique is mostly used for the purpose of effective penetration and delivery of drugs but is also used for the purpose of naturally inducing the death of fat cells by artificially destroying the ion exchange system of the cell membrane.

The non-invasive treatment methods in which electrical pulses are applied locally to the skin as described above are known to be clearly effective in getting rid of a sagging double chin, reducing buccal fat, and improving skin elasticity. In addition, the non-invasive treatment methods have recently gained popularity in the field of skin aesthetics due to having various advantages such as reasonable price, less pain, short treatment time, and a relatively long lasting period of the treatment effect.

Examples of the related art include "Radiofrequency stimulation device" disclosed in Korean Patent Publication No. 10-2015-0049386 (May 8, 2015) and "Hand piece for lipolysis treatment" disclosed in Korean Patent Publication No. 10-2012-0103248 (September 19, 2012). In addition, "Cosmetic medical treatment device" disclosed in Korean Patent Publication No. 10-2020-0042294 (April 23, 2020) is another example of the related art.

In the above examples of the related art, a pair of facing electrodes are basically included as means for applying radiofrequency energy and/or high voltage pulses to the skin of a user. Here, a highly conductive metal material (e.g., copper or nickel) is mostly used as a material of the electrodes, but when the electrodes are only made of a metal, there is a problem in that the performance and durability of the device are degraded due to mechanical properties differing between the electrodes and portions therearound (insulators).

More specifically, when the electrodes are heated due to radiofrequency current or high voltage pulses applied to the electrodes, there is a problem in that, due to a thermal expansion coefficient difference between the heated electrodes and the portions therearound, the electrodes are unable to stably come in close contact with and be fixed to designated positions and are spaced apart from the insulators, and there is a problem in that harmful currents, electric fields, or magnetic fields may be applied to unintended areas due to a clearance of the electrodes caused by such spacing.

Further, when a clearance is formed on the electrodes for the reasons mentioned above, there is a problem in that the electrodes are unable to come in close contact accurately with the skin or an angle of contact with the skin is distorted, and since loss of negative pressure increases in a process in which a treatment target area is locally suctioned using a negative pressure, there is a problem in that a treatment effect may sharply decrease due to treatment being performed in a state in which the treatment target area is not properly lifted to a planned height.

### [Related Art Documents]

(Patent Document 1) Korean Patent Publication No. 10-2015-0049386 (May 8, 2015)
(Patent Document 2) Korean Patent Publication No. 10-2012-0103248 (September 19, 2012)
(Patent Document 3) Korean Patent Publication No. 10-2020-0042294 (April 23, 2020)

### [Disclosure]

### [Technical Problem]

The present invention is directed to providing a cosmetic treatment device that allows electrodes to stably remain in close contact with and fixed to designated positions even when the electrodes are heated due to radiofrequency current or high voltage pulses.

The present invention is also directed to providing a cosmetic treatment device that can prevent or minimize loss of negative pressure that occurs in a process in which a treatment target area is locally suctioned using a negative pressure.

The present invention is also directed to providing a cosmetic treatment device that can suppress or prevent overheating of components disposed inside the device by usefully utilizing discharged air, which has a specific flow speed and a specific flow rate, that is generated in a process of forming a negative pressure.

### [Technical Solution]

According to an embodiment of the present invention, there is provided a cosmetic treatment device including a main body portion having a component mounting space formed therein, a suction treatment portion coupled to the main body portion and configured to suction the skin of a user and apply electrical stimulation thereto, a suction force generator disposed in the component mounting space and configured to generate a suction force and form a negative pressure in a treatment space of the suction treatment portion, and a valve of a solenoid type that is operated to form the negative pressure in the treatment space and release the formed negative pressure, wherein the suction treatment portion includes a suction nozzle made of a resin material and configured to divide the treatment space and a pair of electrodes disposed inside the suction nozzle, and the pair of electrodes have a configuration in which electrode cores made of a resin material are plated with a conductive metal to form a metal conductive layer on a surface of each of the electrode cores.

The cosmetic treatment device according to the present invention may further include a temperature sensor disposed at the suction treatment portion and configured to detect a temperature of the electrodes and a battery disposed in the component mounting space and configured to supply power to the suction force generator, the valve, and the pair of electrodes according to control by a controller.

Here, the pair of electrodes may each include a first surface that is flat and is exposed to the treatment space to directly come into contact with the skin of the user and a second surface coupled to the suction nozzle by insert molding and having protrusions formed thereon.

In addition, a sensor mounting groove or a sensor mounting hole may be formed in one of the pair of electrodes, and at least a portion of the temperature sensor may be disposed to be mounted in the sensor mounting groove or the sensor mounting hole.

Unlike the above, the temperature sensor may be configured as a pair of temperature sensors for one-to-one matching with the pair of electrodes, and in this case, the temperature sensors may each be disposed in a form in which at least a portion of the temperature sensor is mounted in the sensor mounting groove or the sensor mounting hole formed in each of the pair of electrodes.

Preferably, the temperature sensor applied to the present invention may be a negative temperature coefficient (NTC) thermistor having a characteristic in which a resistance value decreases as a temperature of a detection target portion increases.

In addition, the suction force generator may be a diaphragm type vacuum pump that has an air inlet through which air of the treatment space is suctioned and an air outlet through which as much internal air as the suctioned air is discharged, and a plurality of ports communicating with the treatment space may be formed in the suction nozzle of the suction treatment portion.

Here, preferably, the plurality of ports formed in the suction nozzle may consist of an air suction port connected to the air inlet of the suction force generator through a tube and a pressure release port connected to a negative pressure release port of the valve through a tube, and when the air outlet of the suction force generator is disposed to face a circuit board electrically connected to the pair of electrodes, discharged air having a specific flow speed and a specific flow rate that is discharged through the air outlet may be usefully utilized to suppress or prevent overheating of an exothermic element mounted on the circuit board or a substrate thereof.

In addition, a filter in the form of a mesh that has a plurality of through-holes formed therein may be installed at portions of air entrance/exit openings of the ports (the air suction port and the pressure release port) to prevent external foreign matter from being introduced into the device.

In addition, a cover member may be disposed in the treatment space in front of the filter to form a refracted air flow path between an opening of the suction nozzle and the air entrance/exit openings of the ports.

Here, the cover member may consist of a front cover plate disposed in the treatment space while spaced a predetermined distance from the filter to block direct introduction of external foreign matter into the air entrance/exit openings and a rear coupling tube formed at a rear surface of the front cover plate and coupled in a snap fit manner to an inner side of the suction nozzle for the front cover plate to maintain the predetermined distance from the filter in the treatment space.

Preferably, a gap that serves as an entrance of the refracted air flow path may be formed between an outermost surface of the front cover plate and an inner wall surface of the suction nozzle, and a plurality of slit-like air vents may be formed in the rear coupling tube.

### [Advantageous Effects]

According to an embodiment of the present invention, an electrode applying electrical stimulation to the skin has a configuration in which a surface of an electrode core made of an injection-molded resin material is coated with a conductive metal to form a conductive layer. In other words, a base material of the electrode (the electrode core) is made of a material that is the same as or similar to a material of a suction nozzle in which the electrode is embedded. As a result, the variation in deformation caused by heat between the two components (the suction nozzle and the electrode) is insignificant or hardly present.

Accordingly, it is possible to make up for disadvantages of the related art such as an electrode being separated from a suction nozzle or a gap being formed between the electrode and the suction nozzle, and a problem of the related art in which harmful currents, electric fields, or magnetic fields are applied to unintended areas due to the deviation or clearance of the electrode can be addressed. As a result, the durability of the device can be further improved, and good performance of treatment using the device can be maintained for a longer time.

In addition, according to the present invention, since protrusions are formed on one surface of the electrode that is coupled to the suction nozzle by insert molding, at the time of insert molding, an area of the electrode that comes into contact with the suction nozzle can be increased, and a coupling force with the suction nozzle can be significantly improved, and even when a slight gap is formed at a boundary surface between the suction nozzle and the electrode in the insert molding process, loss of negative pressure through the gap can be minimized.

Further, since discharged air having a specific flow speed and a specific flow rate that is generated when a pump (a suction force generator) runs is configured to be utilized to cool highly exothermic components inside the device, overheating of the device can be effectively suppressed or prevented without a separate cooling means such as a cooling fan or a heat sink. In other words, the present invention also has a structural advantage that wind generated when the pump runs can be usefully utilized to prevent overheating of the device.

### [Description of Drawings]

FIG. 1 is a coupling perspective view illustrating a coupling state of a cosmetic treatment device according to an embodiment of the present invention.
FIG. 2 is an exploded perspective view of the cosmetic treatment device illustrated in FIG. 1.
FIG. 3 is a cross-sectional view along line A-A of FIG. 1 of the cosmetic treatment device according to an embodiment of the present invention.
FIG. 4 is a cross-sectional view along line B-B of FIG. 1 of the cosmetic treatment device according to an embodiment of the present invention.
FIG. 5 is a block diagram schematically illustrating a main configuration of the cosmetic treatment device according to an embodiment of the present invention.
FIG. 6 is an enlarged exploded perspective view of a main part of the present invention that illustrates an enlarged view of a suction treatment portion shown in FIG. 2.
FIG. 7 shows an enlarged cross-sectional view and a cut perspective view of a main part of the present invention that illustrate an enlarged view of portion 'C' indicating the suction treatment portion in FIG. 3.
FIG. 8 shows an enlarged cross-sectional view and a cut perspective view of a main part of the present invention that illustrate an enlarged view of portion 'D' indicating the suction treatment portion in FIG. 3.

### [Best Mode of the Invention]

Hereinafter, embodiments of the present invention will be described in detail with reference to the accompanying drawings to allow those of ordinary skill in the art to which the present invention pertains to easily carry out the embodiments of the present invention.

The embodiments which will be described below are provided to more fully describe the present invention to those of ordinary skill in the art, various changes can be made to the following embodiments, and the scope of the present invention is not limited to the following embodiments. On the contrary, these embodiments are provided to make the present disclosure more substantial and complete and to fully convey the idea of the present invention.

Terms used in this specification are used to describe specific embodiments, and are not intended to limit the scope of the present invention. In addition, a singular expression may include a plural expression unless the context clearly indicates otherwise.

Among the terms used in this specification, terms such as "include" or "have" should be understood as specifying that features, numbers, steps, operations, elements, components, or combinations thereof are present and not as precluding the possibility of the presence or addition of one or more other features, numbers, steps, operations, elements, components, or combinations thereof in advance.

In addition, when a certain component is described as being "in front of," "behind," "above," or "below" another component, unless there is some special circumstance, this not only includes a case in which the component is disposed right "in front of," "behind," "above," or "below" the other component, but also includes a case in which another component is disposed between the two components. Further, when a certain component is described as being "connected" to another component, unless there is some special circumstance, this not only includes a case in which the components are directly connected to each other, but also includes a case in which the components are indirectly connected to each other.

In description with reference to the accompanying drawings, the same reference numerals are assigned to the same components, and repeated description thereof will be omitted. Also, in describing the present invention, when detailed description of a known related art is determined as having the possibility of unnecessarily obscuring the gist of the present invention, the detailed description thereof will be omitted.

In addition, the drawings are only for understanding of the idea of the present invention, and the scope of the present invention should not be construed as being limited by the drawings. Further, relative thicknesses, lengths, or sizes in the drawings may be exaggerated for convenience and clarity of description.

A cosmetic treatment device according to an embodiment of the present invention is a device that can help improve skin elasticity by destroying unnecessary fat cells by suctioning the skin locally using a negative pressure and delivering radiofrequency energy and high voltage pulses to the fascial plane. The cosmetic treatment device may be used by a user to directly perform cosmetic treatment on his or her skin or perform cosmetic treatment on a treatment target person.

Hereinafter, exemplary embodiments of the cosmetic treatment device according to the present invention will be described in detail with reference to the accompanying drawings.

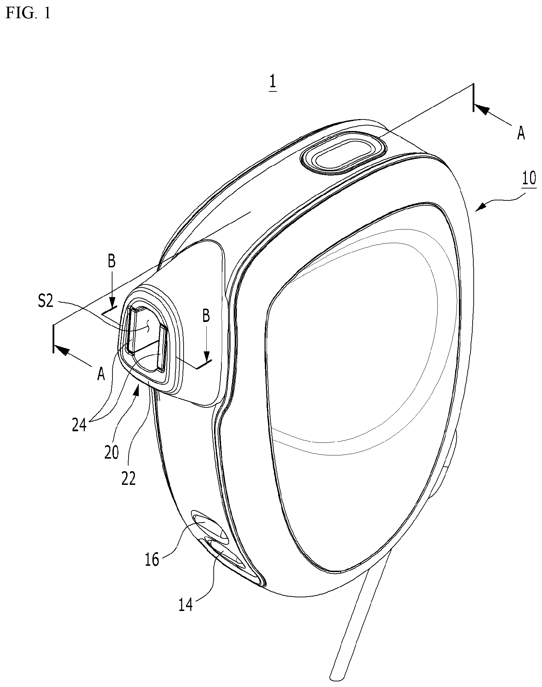

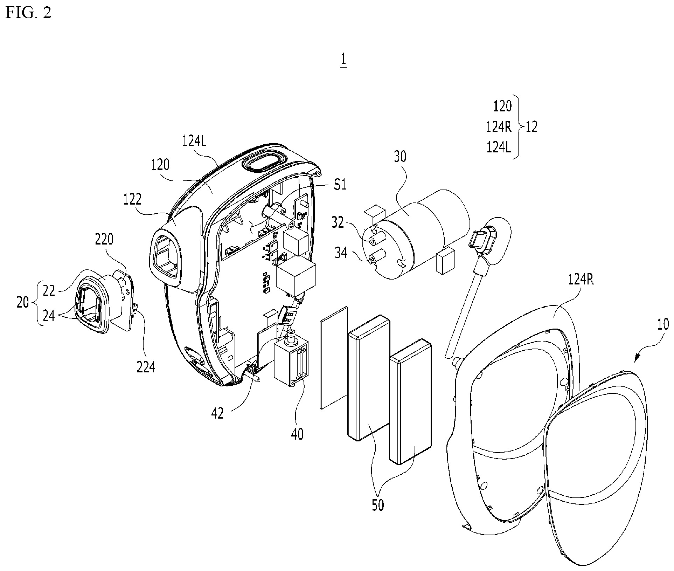

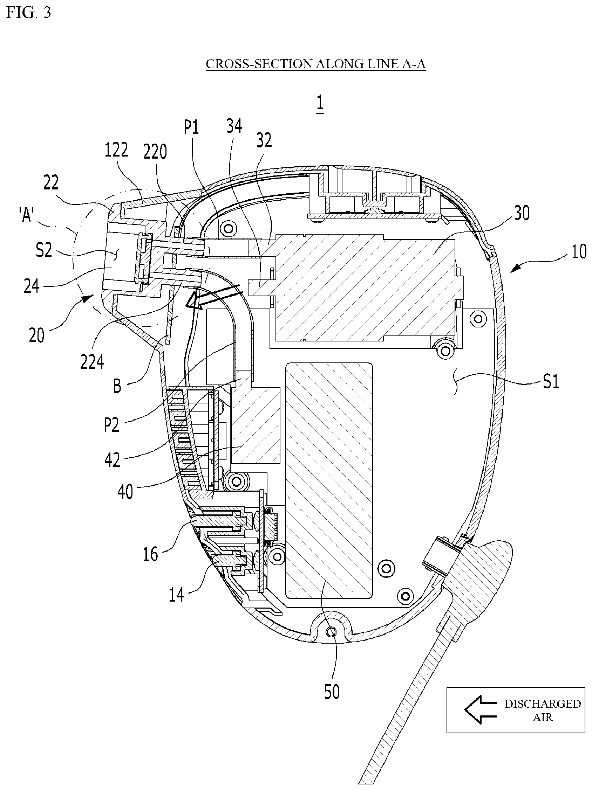

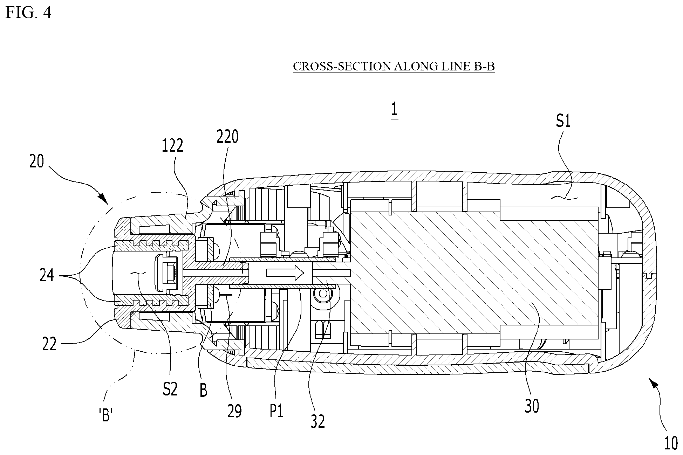

FIG. 1 is a coupling perspective view illustrating a coupling state of a cosmetic treatment device according to an embodiment of the present invention, and FIG. 2 is an exploded perspective view of the cosmetic treatment device illustrated in FIG. 1. In addition, FIGS. 3 and 4 are cross-sectional views for showing an internal configuration and the arrangement relationship of components of the cosmetic treatment device according to an embodiment of the present invention, wherein FIG. 3 is a cross-sectional view along line A-A of FIG. 1 of the cosmetic treatment device according to an embodiment of the present invention, and FIG. 4 is a cross-sectional view along line B-B of FIG. 1 of the cosmetic treatment device according to an embodiment of the present invention.

Referring to FIGS. 1 to 4, a cosmetic treatment device 1 according to an embodiment of the present invention may mainly include a main body portion 10 and a suction treatment portion 20. The main body portion 10 may have a predetermined space (hereinafter referred to as "component mounting space S1"), where components can be mounted, formed therein, and components that operate to allow cosmetic treatment to be performed through the suction treatment portion 20 may be disposed in the component mounting space S1.

The main body portion 10 may include a housing 12 that constitutes an exterior of the device. For example, as illustrated in the exploded perspective view of FIG. 2, the housing 12 may include an intermediate frame 120 having a predetermined thickness and a pair of covers 124L and 126R coupled to both sides of the intermediate frame 120 to form the component mounting space S1 therein together with the intermediate frame 120, but the housing 12 is not limited to the form illustrated in the drawings.

An on/off button 14 by which the power of the device can be turned on or off, a function execution button 16 by which a user can input a substantial device operation command, and the like may be provided on the main body portion 10. Of course, instead of physical buttons, input buttons may be provided in a touch manner in which a touch of a user is recognized and a signal corresponding thereto is output. In addition, an operation display portion (not illustrated) that displays an operation state of the device for the operation state to be recognized by the user may also be provided.

For example, the operation display portion may include a light source and a light guide that output light of different colors according to the operation state of the device, a display, or a combination thereof. When a portion of the housing 12 that covers the operation display portion is plated with tin, the operation display portion may not be exposed to the outside at ordinary times, and the user may check the operation state of the device through the operation display portion only while the device is operating.

In addition to the on/off button 14, the function execution button 16, and the operation display portion mentioned above, a charger connecting terminal (no reference numeral) for charging a battery 50 disposed inside the main body portion 10, a hand strap connecting portion (not illustrated), or the like may be further provided on the main body portion 10. Of course, a new component other than the additional components mentioned above may be added as intended by a designer, or some of the additional components mentioned above may be omitted.

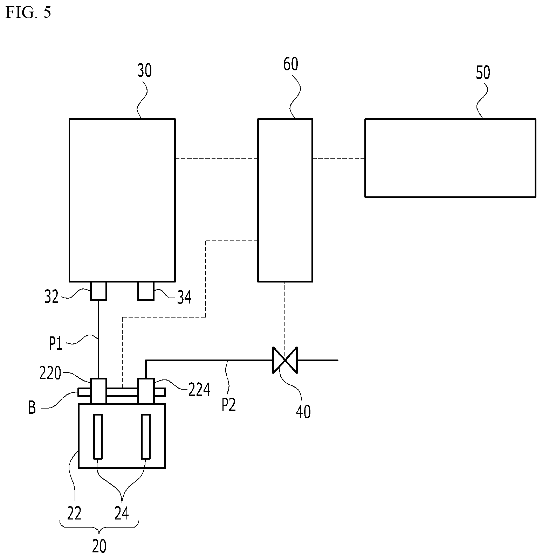

A suction force generator 30, a valve 40 of a solenoid type, the battery 50, a controller 60 (see FIG. 5), etc. may be disposed in the component mounting space S1 of the main body portion 10. Here, the suction force generator 30 may serve to form a negative pressure in a treatment space S2 of the suction treatment portion 20, and the valve 40 may be operated to form the negative pressure in the treatment space S2 and release the formed negative pressure.

The controller 60 includes a control element (not illustrated) such as a microcomputer. The suction force generator 30 and the valve 40 may operate according to a predetermined sequence due to a program set and input to the control element, and due to the set and input program, power may be supplied not only to the control element but also to the suction force generator 30, the valve 40, and a pair of electrodes 24 to be described below that are main components of the suction treatment portion 20.

The suction treatment portion 20 may be coupled to one side of the main body portion 10. For example, the suction treatment portion 20 may be coupled by a structure in which, except for a portion of the suction treatment portion 20, the suction treatment portion 20 is entirely accommodated in a tubular mounting portion 122 provided to protrude outward from one side of the intermediate frame 120 mentioned above (see FIGS. 1 and 2).

In the suction treatment portion 20, a negative pressure may be formed due to action of a suction force generated by the suction force generator 30, and due to the negative pressure, the skin of a treatment target area may be suctioned locally into the treatment space S2 of the suction treatment portion 20 and placed in a raised form. Then, the raised skin comes into contact with the pair of electrodes 24 and receives electrical stimulation in the treatment space S2.

The suction treatment portion 20 includes a suction nozzle 22 configured to divide the treatment space S2 and the pair of electrodes 24 disposed inside the suction nozzle 22. The suction nozzle 22 may be in the form of a vessel that can accurately be engaged with and inserted into the tubular mounting portion 122, and the electrodes 24 may be disposed to face each other while spaced apart from each other inside the suction nozzle 22 and may be disposed on one side of an inner wall of the suction nozzle 22 and the other side of an inner wall of a facing portion so that facing surfaces thereof are exposed to the treatment space S2.

As the facing surfaces (first surfaces 246) of the pair of electrodes 24 are exposed to the treatment space S2, the pair of electrodes 24 may directly come into contact with the skin suctioned upward to the inside (the treatment space S2) of the suction nozzle 22 due to the negative pressure generated by running of the suction force generator and may apply radiofrequency energy for induction of heating of buccal fat and high voltage pulses for electroporation to the skin in contact therewith according to control of the controller 60.

The pair of electrodes 24 may be controlled to, instead of simultaneously applying radiofrequency energy for induction of heating of buccal fat, for example, radiofrequency current of 1 MHz or more, and high voltage pulses for electroporation to the skin suctioned upward to the inside of the suction nozzle 22, apply the radiofrequency energy for induction of heating of buccal fat first and then apply the high voltage pulses for electroporation once it is checked that the corresponding skin is heated to, for example 43 to 45 °C.

By the above-mentioned sequential control (applying radiofrequency energy and then applying high voltage pulses), since a subsequent process (electroporation by the high voltage pulses) is performed in a state in which a support threshold of a cell membrane is lowered due to heating of buccal fat by the radiofrequency energy, electroporation can be implemented more effectively, and as a result, a fat cell destroying effect and a skin elasticity improving effect may be further enhanced.

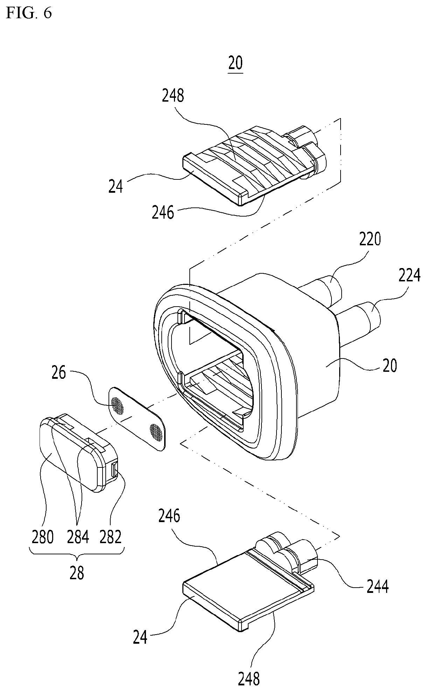

A specific configuration of the suction treatment portion 20 will be described again below with reference to FIGS. 6 to 8.

The suction force generator 30 forming a negative pressure in the suction treatment portion 20 may be a vacuum pump. Among various known types of pumps, preferably, the suction force generator 30 may be a pump that is operated to increase a negative pressure in stages by intermittently suctioning air, for example, a diaphragm type vacuum pump, rather than a typical continuous operation type vacuum pump (e.g., a rotary pump) that is operated to continuously suction air.

When the suction force generator 30 is configured as a diaphragm type vacuum pump, since air and a mechanical device portion are separated by a separator inside the pump, there are also incidental advantages in that there is no concern of contaminating an internal mechanical device with air, and less noise is generated while it is possible to precisely control a fluid flow.

FIG. 5 is a block diagram schematically illustrating a main configuration of the cosmetic treatment device according to an embodiment of the present invention.

Referring to FIG. 5 along with FIGS. 2 to 4 described above, an air inlet 32 and an air outlet 34 may be formed in the suction force generator 30. The suction force generator 30 may suction air of the treatment space S2 by the air inlet 32 being connected to the suction treatment portion 20 through a tube (or hose P1), and air generated by the suction force generator 30 in a process of suctioning the air of the treatment space S2 may be discharged at a specific flow speed and a specific flow rate through the air outlet 34.

Preferably, the air outlet 34 of the suction force generator 30 may be electrically connected to the pair of electrodes 24 described above and may be disposed to face a circuit board B on which highly exothermic components are mounted. Accordingly, the circuit board B or highly exothermic components mounted thereon may be cooled using the air discharged at a specific flow speed and a specific flow rate through the air outlet 34 (refer to a direction of an arrow of "discharged air" of FIG. 3).

A plurality of ports communicating with the treatment space S2 may be formed in the suction nozzle 22 constituting the suction treatment portion 20. The plurality of ports may be an air suction port 220 and a pressure release port 224. The air suction port 220 may be connected to the air inlet 32 through a tube (or hose), and the pressure release port 224 may be connected to a negative pressure release port 42 of the valve 40 through a tube or hose P2.

The valve 40 is disposed in the component mounting space S1 formed inside the main body portion 10 and serves to substantially open or close the pressure release port 224. The valve 40 may be a solenoid valve operated by an electrical signal, and opening/closing control of the pressure release port 224 by the valve 40 may be performed accurately according to a program input to the above-mentioned control element.

The valve 40 may be operated to close the pressure release port 224 while the suction force generator 30 runs. Then, the valve 40 may be switched to open the pressure release port 224 in the last stage (negative pressure release stage) of a series of treatment control processes (negative pressure formation → application of radiofrequency energy through electrodes → application of high voltage pulses through electrodes → negative pressure release) performed by the input program.

Since the pressure release port 224 is closed by the valve 40 while the suction force generator 30 runs, air of the treatment space S2 may be introduced, without great loss, into the suction force generator 30 through the air suction port 220 and the air inlet 32. Accordingly, a negative pressure can be stably formed in the treatment space S2, and as a result, the skin of a treatment target area may be suctioned locally into the treatment space S2 and placed in a form that enables the skin to be treated, that is, a raised form.

When the pressure release port 224 is opened by the valve 40, reversely, outside air is introduced into the treatment space S2 through the pressure release port 224, and the pressure of the treatment space S2 reaches equilibrium with atmospheric pressure. That is, since the negative pressure of the treatment space S2 is released by the outside air introduced through the pressure release port 224 due to opening of the valve 40, the suction nozzle 22 in close contact with the skin may be easily detached from the skin.

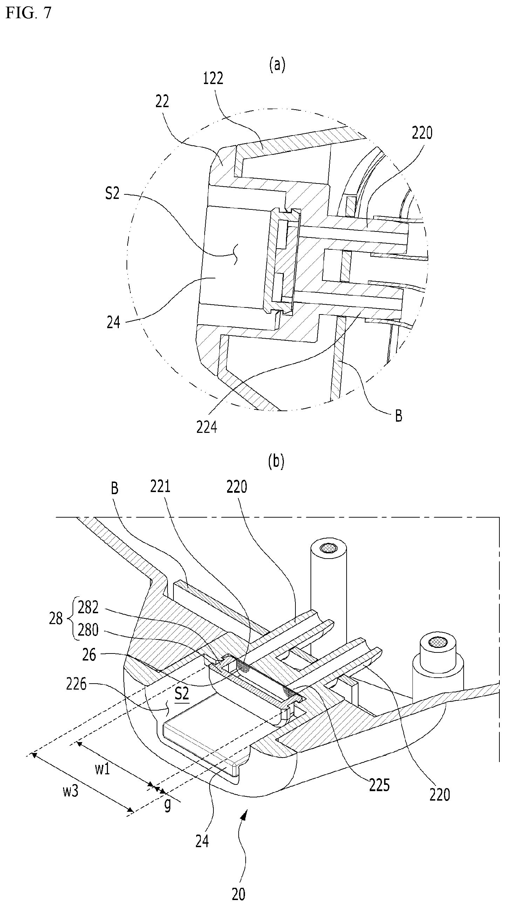

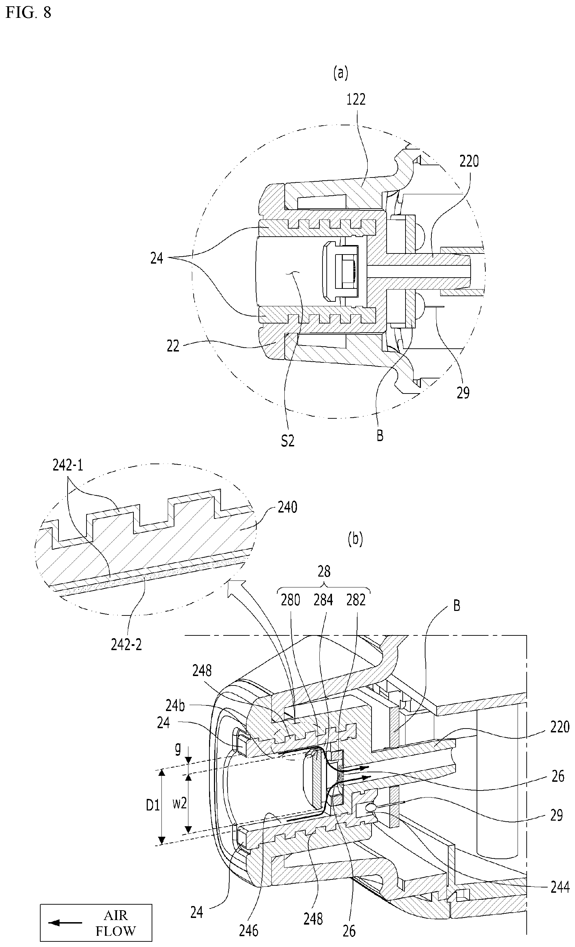

FIGS. 6 to 8 are enlarged views of a main part that illustrate enlarged views of the suction treatment portion, which is the main component of the cosmetic treatment device according to an embodiment of the present invention. FIG. 6 is an enlarged exploded perspective view of the main part of the present invention that illustrates an exploded view of the suction treatment portion, FIG. 7 shows an enlarged cross-sectional view and a cut perspective view of the main part of the present invention that illustrate an enlarged view of portion 'C' of FIG. 3 described above, and FIG. 8 shows an enlarged cross-sectional view and a cut perspective view of the main part of the present invention that illustrate an enlarged view of portion 'D' of FIG. 4 described above.

Referring to FIGS. 6 to 8, due to the action of the suction force generated by the suction force generator 30, a negative pressure may be formed in the suction treatment portion 20 coupled to one side of the main body portion 10. Due to the negative pressure, the skin of the treatment target area may be suctioned locally into the treatment space S2 of the suction treatment portion 20 and placed in a raised form, and the raised skin may come into contact with the pair of electrodes 24 and receive electrical stimulation in the treatment space S2.

The suction treatment portion 20 includes the suction nozzle 22 configured to divide the treatment space S2 and the pair of electrodes 24 disposed inside the suction nozzle 22. For example, the suction nozzle 22 may be substantially configured in the form of a vessel whose cross-section has a circular shape, an angular shape, an elliptical shape, a track shape, or the like, and the pair of electrodes 24 may be disposed in the suction nozzle 22 to face each other while spaced apart from each other in the suction nozzle 22, that is, the treatment space S2.

The pair of electrodes 24 may be disposed through insert molding on one side of an inner wall of the suction nozzle 22 and the other side of an inner wall of a facing portion so that facing surfaces thereof are exposed to the treatment space S2. As the facing surfaces (first surfaces 246) of the pair of electrodes 24 are exposed to the treatment space S2, the skin suctioned upward to the inside (the treatment space S2) of the suction nozzle 22 may directly come into contact with the facing surfaces (first surfaces 246) due to the negative pressure generated by running of the suction force generator.

According to control of the controller 60 (see FIG. 5), the pair of electrodes 24 may apply radiofrequency energy for induction of heating of buccal fat and high voltage pulses for electroporation to the skin that has come into contact therewith after being suctioned upward to the inside (the treatment space S2) of the suction nozzle 22.

The pair of electrodes 24 may be controlled to, instead of simultaneously applying radiofrequency energy for induction of heating of buccal fat, for example, radiofrequency current of 1 MHz or more, and high voltage pulses for electroporation to the skin suctioned upward to the inside of the suction nozzle 22, apply the radiofrequency energy for induction of heating of buccal fat first and then apply the high voltage pulses for electroporation once it is checked that the corresponding skin is heated to, for example 43 to 45 °C.

By the above-mentioned sequential control (applying radiofrequency energy and then applying high voltage pulses), since a subsequent process (electroporation by the high voltage pulses) is performed in a state in which a support threshold of a cell membrane is lowered due to heating of buccal fat by the radiofrequency energy, electroporation can be implemented more effectively, and as a result, a fat cell destroying effect and a skin elasticity improving effect may be further enhanced.

When the electrodes 24 are made of a single metal material, and the suction nozzle 22, which also serves as an insulator, is made of a resin material, a gap may be formed between the electrodes 24 and the suction nozzle 22 due to differences in thermal expansion rate and shrinkage rate between the electrodes 24 and the suction nozzle 22 when the electrodes 24 are heated by the radiofrequency current or high voltage pulses applied thereby and are naturally cooled by air. In addition, a clearance of the electrodes 24 formed as a result may cause harmful currents, electric fields, or magnetic fields to be applied to unintended areas.

Further, when a clearance is formed on the electrodes 24, the electrodes 24 may be unable to come in close contact accurately with the skin or an angle of contact with the skin may be distorted, and since loss of negative pressure increases significantly in a process in which the skin of a treatment target area is locally suctioned using a suction force, a treatment effect may sharply decrease due to treatment being performed in a state in which skin of the treatment target area is not properly lifted to a planned height.

In order to prevent occurrence of such problems caused by a material difference, in an embodiment of the present invention, instead of using the electrodes 24 made of a single metal material, the electrodes 24 each having an electrode core 240 that is injection-molded using a resin and has a surface coated with a conductive metal at a predetermined thickness may be used.

That is, the pair of electrodes 24 applied to the cosmetic treatment device according to an embodiment of the present invention may be components each having the electrode core 240, which is made of a resin material, as a base material and having a metal conductive layer 242 formed thereon by plating the surface of the electrode core 240 with a conductive metal.

Even in forming the metal conductive layer 242 on the surface of the electrode core 240, rather than forming the metal conductive layer 242 using a single material, a configuration in which the surface of the electrode core 240 is plated with nickel (Ni) first to form a nickel-based base layer 242-1, and only the surface (the first surface) of the electrode 24 that is exposed to the treatment space S2 is further plated with titanium 242-2 in consideration of corrosion resistance, biocompatibility, chemical stability, electrical conductivity, etc. may be applied.

The pair of electrodes 24 may include the first surfaces 246 facing each other and second surfaces 248 at the opposite sides. The first surfaces 246 are portions that are exposed to the treatment space S2 and directly come into contact with the skin of a user and may have flat morphology, and the second surfaces 248 are portions coupled to the suction nozzle 22 by insert molding and may have protrusions of regular or irregular patterns or shapes.

When protrusions of regular or irregular patterns or shapes are formed on the second surfaces 248 coupled to the suction nozzle 22 by insert molding, since areas of the electrodes 24 that come into contact with the suction nozzle 22 at the time of insert molding increase, a coupling force with the suction nozzle 22 may significantly increase.

In particular, even when a slight gap is formed at boundary surfaces between the suction nozzle 22 and the electrodes 24 in a process of arranging the electrodes inside the suction nozzle 22 through insert molding, complexity of a pressure loss path increases, and thus loss of negative pressure through the gap can be minimized.

Although the configuration in which protrusions are formed in a concave-convex shape on the second surfaces 248 is illustrated as an example in the drawings, this is only one example, and it should be noted that the protrusions may be changed to have various other forms or patterns such as a waveform in which crests and valleys are continuously formed or a lattice shape.

A temperature sensor 29 (see FIG. 8) may be disposed at the suction treatment portion 20. The temperature sensor 29 may detect temperatures of the electrodes 24 and deliver the detected information to the controller 60, and the controller 60 may perform feedback control for the electrodes 24 based on the temperatures of the electrodes 24 detected by the temperature sensor 29.

For example, when the temperatures of the electrodes 24 detected by the temperature sensor 29 reach a predetermined threshold temperature, the controller 60 may block supply of current to the electrodes 24 to prevent a user from feeling discomfort or getting burned due to high-temperature heat.

The temperature sensor 29 may be disposed to be electrically connected to the controller 60 and have at least a portion mounted in a sensor mounting groove 244 or a sensor mounting hole formed in the electrode 24. The temperature sensor 29 may be disposed only on one of the pair of electrodes 24 or may be disposed on each of the pair of electrodes 24. In this case, the sensor mounting groove 244 or the sensor mounting hole may be formed in each of the pair of electrodes 24.

Although a temperature sensor of any known form or type may be applied as the temperature sensor 29 regardless of a specific operation method, preferably, a temperature sensor of a negative temperature coefficient (NTC) thermistor type (the type having a characteristic that a resistance value decreases as a temperature of a detection target portion increases) that is relatively low-cost, has precise detection sensitivity, and has a rapid response speed may be used.

Meanwhile, a function of preventing introduction of external foreign matter into the device may be applied to the suction nozzle 22 constituting the suction treatment portion 20. Such a function (the function of preventing introduction of external foreign matter into the device) may be achieved by a filter 26 installed to cover an air entrance/exit opening 221 of the air suction port 220 and an air entrance/exit opening 225 of the pressure release port 224 and a cover member 28 configured to block external foreign matter from being directly introduced from the treatment space S2 in front of the filter 26 toward the filter 26.

The filter 26 may be configured in the form of a mesh having a plurality of through-holes of small sizes formed therein, and as mentioned above, the filter 26 may be installed to simultaneously cover the air entrance/exit opening 221 of the air suction port 220 and the air entrance/exit opening 225 of the pressure release port 224. In addition, the cover member 28 may be disposed in the treatment space S2 in front of the filter 26 so that a refracted air flow path (refer to a direction of an arrow of "air flow" of FIG. 8) is formed between an opening 226 of the suction nozzle 22 and the air entrance/exit openings 221 and 225 formed in the air suction port 220 and the pressure release port 224, respectively.

Preferably, the cover member 28 may include a front cover plate 280 disposed in the treatment space S2 while spaced a predetermined distance from the filter 26 to block direct introduction of external foreign matter into the air entrance/exit openings 221 and 225 and a rear coupling tube 282 coupled in a snap fit manner to an inner side of the suction nozzle 22 for the front cover plate 280 to maintain the predetermined distance from the filter 26 in the treatment space S2.

Here, by a horizontal width W1 (see FIG. 7) of the front cover plate 280 being formed to be smaller than a horizontal width W3 (see FIG. 7) of the suction nozzle 22 and a vertical width W2 (see FIG. 8) of the front cover plate 280 being formed to be smaller than a distance D1 (see FIG. 8) between the electrodes 24 (W1<W3 and W2<D1), a gap g that serves as an entrance to the refracted air flow path may be formed between an outermost surface of the front cover plate 280 and an inner wall surface of the suction nozzle 22, and a plurality of slit-like air vents 284 may be formed in the rear coupling tube 282.

According to the configuration described above, among foreign substances introduced in the process of suctioning air of the treatment space S2 according to an operation of the suction force generator 30, foreign substances having relatively large particles may be primarily filtered through the gap g, and foreign substances having relatively small particles, which have passed through the gap g, may be secondarily filtered by the filter 26. That is, since a foreign matter filtering process is performed through primary and secondary filtering, introduction of foreign matter into the device can be surely and reliably prevented.

Further, since the cover member 28 is detachably coupled in a snap fit manner to the inner side of the suction nozzle 22, there is also an advantage in terms of maintenance management in that the filter 26 can be easily cleaned or replaced by separating the cover member 28.

According to an embodiment of the present invention described above, an electrode applying electrical stimulation to the skin has a configuration in which a surface of an electrode core made of an injection-molded resin material is coated with a conductive metal to form a conductive layer. In other words, a base material of the electrode (the electrode core) is made of a material that is the same as or similar to a material of a suction nozzle in which the electrode is embedded. As a result, the variation in deformation caused by heat between the two components (the suction nozzle and the electrode) is insignificant or hardly present.

Accordingly, it is possible to make up for disadvantages of the related art such as an electrode being separated from a suction nozzle or a gap being formed between the electrode and the suction nozzle, and a problem of the related art in that harmful currents, electric fields, or magnetic fields are applied to unintended areas due to the deviation or clearance of the electrode can be addressed. As a result, the durability of the device can be further improved, and good performance of treatment using the device can be maintained for a longer time.

In addition, according to the present invention, since one surface of the electrode that is coupled to the suction nozzle by insert molding is configured to have a waveform or a concave-convex shape, at the time of insert molding, an area of the electrode that comes into contact with the suction nozzle can be increased, and a coupling force with the suction nozzle can be significantly improved, and even when a slight gap is formed at a boundary surface between the suction nozzle and the electrode in the insert molding process, loss of negative pressure through the gap can be prevented or minimized.

Further, since discharged air having a specific flow speed and a specific flow rate that is generated when a pump (a suction force generator) runs is configured to be utilized to cool highly exothermic components inside the device, overheating of the device can be effectively suppressed or prevented without a separate cooling means such as a cooling fan or a heat sink. In other words, the present invention also has a structural advantage that wind generated when the pump runs can be usefully utilized to prevent overheating of the device.

In the detailed description of the present invention given above, only some embodiments according to the present invention have been described. The present invention should not be understood as being limited to the embodiments mentioned in the detailed description above, and on the contrary, the present invention should be understood as including all modifications, equivalents, and substitutes within the spirit and scope of the present invention defined by the appended claims.

**[Description of reference numerals]**

| | | | |
|---|---|---|---|
| 1: | cosmetic treatment device | | |
| 10: | main body portion | 12: | housing |
| 14: | on/off button | 16: | function execution button |
| 20: | suction treatment portion | 22: | suction nozzle |
| 24: | electrode | 26: | filter |
| 28: | cover member | 30: | suction force generator |
| 32: | air inlet | 34: | air outlet |
| 40: | valve | 42: | negative pressure release port |
| 50: | battery | 60: | controller |
| 120: | intermediate frame | 122: | tubular mounting portion |
| 220: | air suction port | 224: | pressure release port |
| 240: | electrode core | 242: | metal conductive layer |
| 244: | sensor mounting groove | 246: | first surface of electrode |
| 248: | second surface of electrode | | |
| 280: | front cover plate | 282: | rear coupling tube |
| 284: | air vent | | |
| D1: | distance between electrodes | g: | gap |
| P1, P2: | tube (or hose) | | |
| S1: | component mounting space | S2: | treatment space |
| W1: | horizontal width of front cover plate | | |
| W2: | vertical width of front cover plate | | |
| W3: | horizontal width of suction nozzle | | |

### [Modes of the Invention]

The relevant content has been described above in the best mode of the invention.

### [Industrial Applicability]

The present invention can provide a cosmetic treatment device that improves skin elasticity by destroying unnecessary fat cells by suctioning the skin using a negative pressure and delivering radiofrequency energy and high voltage pulses to the fascial plane.

## Claims

1. A cosmetic treatment device comprising:
a main body portion; and
a suction treatment portion coupled to the main body portion and having a treatment space formed therein,
wherein the suction treatment portion includes a suction nozzle made of a resin material and configured to divide the treatment space and a pair of electrodes disposed inside the suction nozzle, and
the pair of electrodes have a configuration in which electrode cores made of a resin material are plated with a conductive metal to form a metal conductive layer on a surface of each of the electrode cores.

2. A cosmetic treatment device comprising:
a main body portion; and
a suction treatment portion coupled to the main body portion and having a treatment space formed therein,
wherein the suction treatment portion includes a suction nozzle configured to divide the treatment space and a pair of electrodes disposed inside the suction nozzle, and
the pair of electrodes each include a first surface that is flat and is exposed to the treatment space to directly come into contact with the skin of a user and a second surface coupled to the suction nozzle by insert molding and having protrusions formed thereon.

3. The cosmetic treatment device of claim 1 or 2, further comprising:
a suction force generator disposed in the main body portion and configured to generate a suction force and form a negative pressure in the treatment space; and
a valve operated to form the negative pressure in the treatment space and release the formed negative pressure.

4. The cosmetic treatment device of claim 3, wherein:
the suction force generator is a diaphragm type vacuum pump that has an air inlet through which air of the treatment space is suctioned and an air outlet through which as much internal air as the suctioned air is discharged; and
the suction nozzle has a plurality of ports communicating with the treatment space.

5. The cosmetic treatment device of claim 4, wherein the air outlet is disposed to discharge air toward a circuit board electrically connected to the pair of electrodes.

6. The cosmetic treatment device of claim 4, wherein the plurality of ports include:
an air suction port connected to the air inlet of the suction force generator through a tube; and
a pressure release port connected to a negative pressure release port of the valve through a tube.

7. The cosmetic treatment device of claim 1 or 2, further comprising a temperature sensor disposed at the suction treatment portion and configured to detect temperatures of the electrodes.

8. The cosmetic treatment device of claim 7, wherein:
a sensor mounting groove or a sensor mounting hole is formed in one of the pair of electrodes; and
at least a portion of the temperature sensor is disposed to be mounted in the sensor mounting groove or the sensor mounting hole.

9. The cosmetic treatment device of claim 7, wherein:
the temperature sensor is configured as a pair of temperature sensors for one-to-one matching with the pair of electrodes; and
the temperature sensors are each disposed in a form in which at least a portion of the temperature sensor is mounted in the sensor mounting groove or the sensor mounting hole formed in each of the pair of electrodes.

10. The cosmetic treatment device of claim 7, wherein the temperature sensor is a negative temperature coefficient (NTC) thermistor having a characteristic in which a resistance value decreases as a temperature of a detection target portion increases.

11. The cosmetic treatment device of claim 1 or 2, wherein:
the suction nozzle includes a plurality of ports communicating with the treatment space; and
a filter in the form of a mesh that has a plurality of through-holes formed therein is installed to cover air entrance/exit openings of the ports.

12. The cosmetic treatment device of claim 10, wherein a cover member is further provided in the treatment space in front of the filter to form a refracted air flow path between an opening of the suction nozzle and the air entrance/exit openings of the ports.

13. The cosmetic treatment device of claim 11, wherein the cover member includes:
a front cover plate disposed in the treatment space while spaced a predetermined distance from the filter to block direct introduction of external foreign matter into the air entrance/exit openings; and
a rear coupling tube formed at a rear surface of the front cover plate and coupled in a snap fit manner to an inner side of the suction nozzle.

14. The cosmetic treatment device of claim 13, wherein:
a gap that serves as an entrance of the refracted air flow path is formed between an outermost surface of the front cover plate and an inner wall surface of the suction nozzle; and
a plurality of slit-like air vents are formed in the rear coupling tube.
